# EUROPÄISCHE PATENTSCHRIFT

(11) **EP 2 631 201 B1**
(45) Veröffentlichungstag und Bekanntmachung des Hinweises auf die Patenterteilung: **18.06.2014**
(21) Anmeldenummer: 12156529.5
(22) Anmeldetag: 22.02.2012
(51) Int. Cl.: B65G 1/04, B65G 1/137

(54) **Verfahren zum Ermitteln von Fehllagerungen von Arzneimittelpackungen**
Method for determining when medicine packages are positioned incorrectly
Procédé pour déterminer le mauvais positionement de médicaments

(43) Veröffentlichungstag der Anmeldung: 28.08.2013
(73) Patentinhaber: CareFusion Germany 326 GmbH, 53539 Kelberg (DE)
(72) Erfinder: Hellenbrand, Christoph, 56761 Kaifenheim (DE); Klapperich, Andreas, 56745 Rieden (DE); Reif, Dennis, 56759 Kaisersesch (DE)
(74) Vertreter: Zenz

(56) Entgegenhaltungen:
- EP-A1- 1 627 830
- CN-U- 201 506 600
- DE-U1-202004 004 292
- JP-A- 6 024 528

## Beschreibung

Die vorliegende Erfindung betrifft ein Verfahren zum Betreiben einer Apothekenkommissioniervorrichtung und insbesondere betrifft die vorliegende Erfindung ein Verfahren zum Ermitteln und ggf. Beheben von Fehllagerungen von Arzneimittelpackungen in einer Apothekenkommissioniervorrichtung mit zumindest einer Regalreihe mit jeweils einer Mehrzahl von sich in einer horizontalen Richtung (X-Achse) erstreckenden Regalböden und einer Mehrzahl von sich in einer vertikalen Richtung erstreckenden Regalwänden, wobei die Regalböden und Regalwände eine Mehrzahl von Regalfächern bilden, zumindest einem vor der Regalreihe horizontal und vertikal verfahrbaren Bediengerät, wobei das Bediengerät eine Greifvorrichtung zum Ein- und/oder Auslagern von Arzneimittelpackungen auf die bzw. von den Regalböden sowie einen Sensor umfasst, und einer mit dem Bediengerät gekoppelten Steuereinrichtung.

Bei modernen Apothekenkommissioniervorrichtungen wird eine große Anzahl verschiedener und unterschiedlich dimensionierter Arzneimittelpackungen (Stückgüter) chaotisch und platzoptimiert auf langgestreckten Regalböden gelagert. Diese bilden zusammen mit den Regalwänden die Regalfächer, wobei pro Regalfach, d. h. pro Regalboden, eine große Anzahl von Arzneimittelpackungen gelagert wird.

Bei der Einlagerung von Arzneimittelpackungen in ein Regalfach wird jeder Arzneimittelpackung ein Lagerplatz zugewiesen und dieser, mit anderen Informationen zu einer Arzneimittelpackung, in einer Steuereinrichtung gespeichert. Für eine ggf. zu erfolgende Aus- oder Umlagerung einer bestimmten Arzneimittelpackung kann mit Hilfe der in der Steuereinrichtung gespeicherten Informationen mit dem Bediengerät auf die Arzneimittelpackung zugegriffen werden.

Die Ein- oder Auslagerung verläuft üblicherweise vollkommen automatisch, ohne dass ein Benutzer, außer durch Eingabe verschiedener Anweisungen, eingreifen muss. Kommt es jedoch zu Störungen bei der Apothekenkommissioniervorrichtung, wie beispielsweise einem Stromausfall, einem mechanischen oder elektrischen Defekt, muss ein Benutzer die Auslagerung der benötigten Arzneimittelpackungen händisch vornehmen, wobei der Benutzer über eine Benutzerführung der Apothekenkommissioniervorrichtung zu dem entsprechenden Regalfach geleitet wird, bei welchem die zu entnehmende Arzneimittelpackung gelagert ist.

Bei der händischen Auslagerung können diverse Fehler auftreten; beispielsweise kann die falsche Arzneimittelpackung entnommen werden, bei dem Entnehmen benachbarte Arzneimittelpackungen verschoben oder eine Arzneimittelpackung, die der zu entnehmenden im Wege liegt, in ein anderes Regalfach umgelagert und dort vergessen werden. Dies führt dazu, dass Fehllagerungen von Arzneimittelpackungen in dem händisch bedienten Regalfach (und ggf. weiteren Fächern, falls Arzneimittelpackungen umgelagert wurden) auftreten können. Wenn nach der Behebung der Störung die Apothekenkommissioniervorrichtung wieder auf vollautomatischen Betrieb umgestellt wird, kann es in Abhängigkeit von der Art der Fehllagerungen dazu kommen, dass Arzneimittelpackungen aus Regalfächern mit Fehllagerungen nicht oder zumindest nur mit Verzögerung entnommen werden können.

Das Dokument JP 6 024 528 A ein Verfahren zum Erkennen von Raum in einer Apothekenkommissioniervorrichtung mit zumindest einer Regalreihe mit jeweils einer Mehrzahl von sich in einer horizontalen Richtung erstreckenden Regalböden und einer Mehrzahl von sich in einer vertikalen Richtung ersteckenden Regalwänden, wobei die Regalböden und die Regalwände (12) eine Mehrzahl von Regalfächern bilden, zumindest einem vor der Regalreihe horizontal und vertikal verfahrbaren Bediengerät, wobei das Bediengerät eine Greifvorrichtung zum Einlagern von Arzneimittelpackungen auf die Regalböden sowie einen Sensor umfasst,
und einer mit dem Bediengerät gekoppelten Steuereinrichtung, wobei zum Erkennen von Raum :
- das Bediengerät an eine vorgegebene Regalposition bei einem ermittelten Regalfach verfahren wird,
- mit dem Sensor die Einlagerungstiefe bei verschiedenen X-Positionen ermittelt wird.

In diesem System wird das Bediengerät benutzt, um die Möglichkeit der Einlagerung von einem zusätzlichen Artikel in einen besonderen Regalboden zu bewerten.

Dieses System erlaubt jedoch nicht Fehllagerungen zu erkennen.

Es ist daher Aufgabe der vorliegenden Erfindung, ein Verfahren zum Ermitteln und ggf. Beheben von Fehllagerungen von Arzneimittelpackungen in einer Apothekenkommissioniervorrichtung bereitzustellen.

Diese Aufgabe wird erfindungsgemäß durch ein Verfahren mit den Merkmalen von Patentanspruch 1 gelöst.

Die bei dem erfindungsgemäßen Verfahren verwendete Apothe-kenkommissioniervorrichtung umfasst zumindest eine Regalreihe mit jeweils einer Mehrzahl von sich in einer horizontalen Richtung (X-Richtung, X-Achse) erstreckenden Regalböden und einer Mehrzahl von sich in einer vertikalen Richtung ersteckenden Regalwänden, wobei die Regalböden und die Regalwände eine Mehrzahl von üblicherweise langgestreckten (in X-Richtung)Regalfächern bilden. Ferner umfasst die Apothekenkommisioniervorrichtung eine vor der Regalreihe horizontal und vertikal verfahrbares Bediengerät und eine mit dem Bediengerät gekoppelte Steuereinrichtung, wobei das Bediengerät eine Greifvorrichtung zum Ein- und/oder Auslagern von Arzneimittelpackungen auf die bzw. von den Regalböden und einen Sensor umfasst.

Im Rahmen dieser Anmeldung ist der Begriff "Arzneimittelpackung" als "zumindest eine Arzneimittelpackungen" auszulegen; die Auslagerung einer Mehrzahl (hintereinander) eingelagerter Arzneimittelpackungen soll auch umfasst sein.

Unter dem Begriff des Sensors ist im Rahmen dieser Anmeldung ein Abstandssensor mit einer Einrichtung zum Aussenden von Messstrahlen und zum Empfangen von Messstrahlen zu verstehen, wobei die beiden Einrichtungen in einer Einrichtung kombiniert sein können. Bei der Abstandsbestimmung kann der Sensor Richtung oder Laufzeit eines aktiv ausgesandten, an einer Oberfläche (einer Arzneimittelpackung oder eines Bauteils der Apothekenkommisioniervorrichtung) reflektierten Signales detektieren. Die Abstandsbestimmung kann aber auch kapazitiv oder anhand der Parallaxe bzw. eines Stereobildes einer elektronischen Kamera durchgeführt werden.

Bei Apothekenkommissioniervorrichtungen sind Laserabstandssensoren, die nach dem Prinzip der Lasertriangulation arbeiten, üblich. Dabei wird ein Laserstrahl auf eine Oberfläche (s.o.) gerichtet und mit der im Sensor befindlichen Empfangseinrichtung (beispielsweise Kamera, Photo-Diode, Dioden-Array) beobachtet. Ändert sich die Entfernung der Oberfläche vom Sensor, ändert sich auch der Winkel, unter dem der Lichtpunkt beobachtet wird und damit die Position des Abbildes auf dem Empfänger der Empfangseinrichtung. Wird beispielsweise ein Dioden-Array verwendet ändert sich mit dem Abstand die Diode, welche den reflektierten Lichtstrahl detektiert. Aus der Positionsänderung kann die Entfernung der Oberfläche vom Sensor berechnet werden.

Aus Kostengründen wird die Abstandsmessung regelmäßig als "Einzelpunktmessung" durchgeführt, d.h. der Messstrahl wird stets in einem konstanten Winkel ausgesendet und trifft, ohne Bewegung des Bediengeräts, stets auf dem gleichen Punkt auf (Einzelpunkt). Der reflektierte Strahl wird üblicherweise mit einem Dioden-Array detektiert.

Die Abstandsmessung kann dabei so durchgeführt werden, dass lediglich die Detektion bei einer bestimmten Diode (oder einem

Dioden-Bündel) des Arrays von der Detektion außerhalb dieser Diode bzw. des Dioden-Bündels unterschieden wird. Die Abstandsmessung kennt dann lediglich zwei Zustände (nachfolgend "binäre Abstandsmessung").

Zum Erkennen von Fehllagerungen werden zunächst Regalfächer mit möglichen Fehllagerungen von Arzneimittelpackungen ermittelt, beispielsweise indem diese von einem Benutzer als solche markiert werden. Da insbesondere bei einer händischen Entnahme die Gefahr besteht, dass Arzneimittelpackungen beispielsweise verschoben werden, sollte jedes Regalfach, in welches händisch eingegriffen wurde, markiert werden. Dann wird das Bediengerät an eine vorgegebene Regalposition bei einem ermittelten Regalfach verfahren, wobei es sich bei dieser Regalposition regelmäßig um die linke oder rechte Ecke (bezogen auf die Ecke Regalboden/Regalwand) handeln wird, um die nachfolgenden Schritte des Verfahrens zu begünstigen.

Mit dem Bediengerät wird bei eingeschaltetem Sensor, beginnend bei der angefahrenen Regalposition, das Regalfach in horizontaler Richtung (X-Achse) abgefahren und mit dem Sensor die Einlagerungstiefe bei verschiedenen X-Positionen ermittelt. In welchem Umfang und an welchen bzw. wie vielen Positionen die Einlagerungstiefe bestimmt wird ergibt aus der Art des verwendeten Sensors und damit der Art und Weise, wie die Einlagerungstiefe bestimmt wird. Wird beispielsweise ein Sensor zur Einzelpunktmessung verwendet, muss das Regalfach zumindest in dem Bereich, in welchem eine Fehllagerung auftreten kann, vollständig in horizontaler Richtung abgefahren werden. Wird beispielsweise ein Sensor mit einer Kamera verwendet, kann es genügen, bei einer von der Breite des Regalfachs abhängigen Zahl von X-Positionen die Einlagerungstiefe zu ermitteln, wobei dann keine punktuelle Einlagerungstiefe, sondern die Einlagerungstiefe für einen ganzen Abschnitt bestimmt wird.

Auf der Basis der ermittelten Einlagerungstiefen wird ein virtuelles Istabbild des Regalfaches erstellt, wobei zum Erstellen dieses Istabbilds auf die von dem Sensor ermittelten Daten zurückgegriffen wird. Das so erstellte Istabbild (AB_{I}) wird mit einem Sollabbild (AB_{S}) des Regalfaches verglichen und Abweichungen zwischen dem Sollabbild und dem Istabbild werden ausgewertet. Sollten mehrere Regalfächer als Regalfächer mit möglichen Fehllagerungen markiert sein, wird dieser Vorgang für die verbliebenen Regalfächer wiederholt.

Auf der Basis der Auswertung der Abweichungen zwischen dem Sollabbild eines Regalfaches und dem Istabbild lässt sich auf eine Fehllagerung in dem Regalfach schließen; dazu wird auf die mit dem Sensor ermittelbare Einlagerungstiefe bei verschiedenen X-Positionen eines Regalfaches zurückgegriffen. Ist bei einer beliebigen X-Position eine Arzneimittelpackung gelagert (üblicherweise in Anlage an die Regalrückwand), so ist die Einlagerungstiefe eine andere, als wäre keine oder mehrere Arzneimittelpackungen eingelagert. Die für eine bestimmte X-Position erwartete Einlagerungstiefe ist bekannt, da mit Einlagerung jeder Arzneimittelpackung deren Abmessungen gespeichert werden; diese Informationen sind für die platzoptimierte Lagerung der Arzneimittelpackungen unbedingt notwendig.

Bei dem erfindungsgemäßen Verfahren wird also lediglich auf bekannte Informationen zurückgegriffen; eine Speicherung von zusätzlichen Informationen ist nicht erforderlich. Das Verfahren lässt sich mit den "üblichen" Bauteilen durchführen, eine Verbauung zusätzlicher Bauteile ist nicht notwendig. Das erfindungsgemäße Verfahren lässt sich somit kostengünstig und rasch implementieren.

Regalfächer mit möglichen Fehllagerungen können ermittelt werden, indem diese von einem Benutzer oder automatisch nach einer händischen Auslagerung von Arzneimittelpackungen als solche markiert werden. Eine entsprechende Markierung von Regalfächern beschränkt die Anzahl der zu untersuchenden Fächer; die Abarbeitungszeit des Verfahrens sinkt.

Nach einer Störung der Apothekenkommisioniervorrichtung können vor der Störung betroffene Regalfächer (dies können auch sämtliche Regalfächer sein) als Regalfächer mit möglichen Fehllagerungen markiert werden. Dies ist insbesondere dann angezeigt, wenn es zu Aus- bzw. Einlagerungen gekommen sein kann, die nicht in dem theoretischen Lagerbestand vermerkt wurden (beispielsweise aufgrund einer Fehlfunktion des Bediengeräts oder der Identifiziereinrichtung). Sämtliche Regalfächer für eine Überprüfung zu markieren ist auch dann sinnvoll, wenn eine "In-ventur" durchgeführt werden soll.

Das Sollabbild eines Regalfaches kann bei einer bevorzugten Ausführungsform des Verfahrens für den Vergleich mit dem erstellten Istabbild anhand der Sollbelegung des Regalfaches errechnet werden. Die Steuereinrichtung speichert bei der Einlagerung von Arzneimittelpackungen sämtliche Informationen (u.a. Ablageplatz, Abmessungen), die für die Berechnung eines Sollabbilds notwendig sind. Das Berechnen des Sollabbildes "on demand" hat den Vorteil, dass die notwendige Rechenleistung erst dann abgerufen wird, wenn sie wirklich benötigt wird. Alternativ kann das Sollabbild des Regalfaches beim Ein- und/oder Auslagern von Arzneimittelpackungen in bzw. aus dem Regalfaches erstellt bzw. aktualisiert werden, in einem Speicher der Steuereinrichtung gespeichert werden und für den Vergleich aus einem Speicher der Steuereinrichtung geladen werden. Diese alternative Ausführungsform hat den Vorteil, dass das Erkennen besonders rasch durchgeführt werden kann, da das Sollabbild nicht errechnet werden muss, sondern bereits in dem Speicher der Steuereinrichtung vorhanden ist.

Wenn beispielsweise bei einem Systemausfall des Bediengerätes die automatische Auslagerung nicht möglich ist, müssen Arzneimittelpackungen ggf. von dem Benutzer händisch ausgelagert werden. Dazu kann er beispielsweise über eine entsprechende Menüführung zu dem Regalfach und dem Lagerplatz der Arzneimittelpackung geführt werden. Bei der automatischen Auslagerung wird die Arzneimittelpackung automatisch aus dem theoretischen Lagerbestand gelöscht und der Lagerplatz für eine erneute Einlagerung freigegeben (der Lagerplatz wurde bei einer Einlagerung der ausgelagerten Arzneimittelpackung für eine weitere Einlagerung gesperrt) Aufgrund des Zeitdrucks kann es bei einer händischen Auslagerung vorkommen, dass der Benutzer die Quittierung der Arzneimittelpackung vergisst; der theoretische Lagerbestand sowie der theoretisch freie Lagerplatz weichen dann von dem praktischen ab. Um dies zu vermeiden wird bei einer bevorzugten Ausführungsform, wenn die Auswertung der Abweichungen zwischen dem Sollabbild und dem Istabbild ergibt, dass eine Arzneimittelpackung in dem Regalfach fehlt, diese aus einem in der Steuereinrichtung gespeicherten theoretischen Lagerbestand gelöscht.

Bei der händischen Entnahme kann es auch vorkommen, dass ein Bediener zur Auslagerung einer bestimmten Arzneimittelpackung zunächst eine andere Arzneimittelpackung umlagern muss und diese Packung dann in dem Regalfach, in welche sie umgeräumt wurde, verbleibt; in diesem Regalfach befindet sich dann eine Arzneimittelpackung "zu viel", die nicht im theoretischen Lagerbestand für diese Regalfach verzeichnet ist. Bei einer bevorzugten Ausführungsform wird, wenn die Auswertung der Abweichungen zwischen dem Sollabbild und dem Istabbild ergibt, dass eine nicht im theoretischen Lagerbestand verzeichnete Arzneimittelpackung in dem Regalfach gelagert ist, diese ausgelagert oder identifiziert und erneut eingelagert.

Bei der händischen Entnahme einer Packung kann es aufgrund der Nähe der Packungen zueinander vorkommen, dass eine oder mehrere benachbarte Arzneimittelpackungen verschoben werden, diese also nicht mehr so liegen, wie es entsprechend der Einlagerung erwartet wird. Im Rahmen dieser Anmeldung soll der Begriff "Verschoben" nicht nur parallel zu einer Achse bewegte Arzneimittelpackungen umfassen, sondern auch solche die nur oder auch verdreht (um die Zentralachse auf einem Regalboden) sind.

Anhand der Fehllagerung der Arzneimittelpackung(en) wir dann bei einem bevorzugten Ausführungsbeispiel geprüft, ob die verschobene Arzneimittelpackung mit der Greifvorrichtung ausgelagert werden kann. Wenn dies möglich ist, was insbesondere von der Schieflage der Arzneimittelpackung und der Packungsdichte auf dem Regalboden abhängt, wird diese Arzneimittelpackung ausgelagert und erneut eingelagert wird.

Wenn eine Auslagerung mit der Greifvorrichtung nicht möglich ist (beispielweise aufgrund der zu geringen Beabstandung zu benachbarten Arzneimittelpackungen) wird zunächst geprüft, ob die Arzneimittelpackung eindeutig einer theoretisch vorhandenen Arzneimittelpackung zugeordnet werden kann. Ist dies der Fall, d.h. ist bekannt, um welche Packung es sich handelt, kann aber nicht ohne Störung der benachbarten Arzneimittelpackungen ausgelagert werden, werden zunächst bei einer Ausführungsform die umliegenden Arzneimittelpackungen ausgelagert (bis auf die Arzneimittelpackung mit Fehllagerung) auf welche ohne Störung der benachbarten Packungen zugegriffen werden kann, und dann wird die verbliebende Arzneimittel ausgelagert und erneut eingelagert.

Wenn die Arzneimittelpackung nicht eindeutig einer theoretisch vorhandenen Arzneimittelpackung zugeordnet werden kann, wird bei einer Ausführungsform zunächst (anhand des Istabbilds, des Sollabbilds und der theoretischen Belegung) ermittelt, welche benachbarten Arzneimittelpackungen theoretisch vorhandenen Arzneimittelpackungen zugeordnet werden können (und auch ausgelagert werden können). Dann werden all jene Arzneimittelpackungen, die theoretisch vorhandenen Arzneimittelpackungen zugeordnet werden können, ausgelagert. Im Anschluss werden die verbliebenen Arzneimittelpackungen ausgelagert, erneut identifiziert und dann wieder eingelagert. Bei diesem Ausführungsbeispiel ist es aufgrund der fehlenden Möglichkeit der Zuordnung verschobener Arzneimittelpackungen notwendig, diese vor der erneuten Einlagerung zu identifizieren. Bei sämtlichen der vorgenannten Ausführungsbeispiele geht das Aus-, Um- und Wiedereinlagern natürlich mit einer stetigen Aktualisierung der theoretischen Belegung einher.

Stellt sich beispielsweise bei der Auswertung der Abweichungen heraus, dass Arzneimittelpackungen nicht mit der Greifvorrichtung ausgelagert werden können, wird der Benutzer entsprechend informiert und zur händischen Auslagerung aufgefordert.

Bei einem bevorzugten Ausführungsbeispiel wird das Regalfach nach der Bereinigung der Fehllagerungen noch einmal geprüft und bei positivem Ergebnis dieser Prüfung (alle Arzneimittelpackungen sind an dem theoretisch vorgesehenen Lagerplatz gelagert) wird das Regalfach zur weiteren Ein-/Auslagerung freigegeben. Dazu wird das Regalfach in horizontaler Richtung (X-Achse) noch einmal vollständig abgefahren und mit dem Sensor wird die Einlagerungstiefe bei verschiedenen X-Positionen ermittelt. Auf der Basis der ermittelten Einlagerungstiefen wird ein virtuelles Istabbild des Regalfaches erstellt und das erstellte Istabbild mit einem Sollabbild des Regalfaches verglichen und Abweichungen zwischen dem Sollabbild und dem Istabbild werden ausgewertet. Sofern das Regalfach zuvor für eine weitere Einlagerung gesperrt war, wird es bei einer positiven Auswertung wieder zur Einlagerung freigegeben.

Im Nachfolgenden wird das erfindungsgemäße Verfahren anhand von in der Zeichnung veranschaulichten bevorzugten Ausführungsformen näher beschrieben. In der Zeichnung zeigen bzw. zeigt:
Figur 1 eine Seitenansicht einer Regalreihe einer Apothekenkommissioniervorrichtung;
Figur 2a eine Draufsicht auf ein schematisch dargestelltes Regalfach sowie eine schematische Darstellung des vor dem Regalfach verfahrbaren Bediengeräts;
Figur 2b eine Vorderansicht des in Figur 2a dargestellten Regalfachs;
Figur 2c das mit dem Sensor ermittelte Istabbild des in Figur 2a dargestellten Regalfaches im Vergleich mit dem berechneten Sollabbild;
Figur 3a eine Draufsicht auf ein Regalfach mit Fehlbelegung;
Figur 3b einen Vergleich zwischen dem Sollabbild gemäß Figur 2a und dem Istabbild des in Figur 3a gezeigten Regalfachs;
Figur 4a eine Draufsicht auf ein Regalfach mit Fehlbelegung;
Figur 4b einen Vergleich zwischen dem Sollabbild gemäß Figur 2a und dem Istabbild des in Figur 4a gezeigten Regalfaches;
Figur 5a eine Draufsicht auf ein Regalfach mit Fehlbelegung;
Figur 5b einen Vergleich zwischen dem Sollabbild gemäß Figur 2a und dem Istabbild des in Figur 5a gezeigten Regalfachs;
Figur 6a eine Draufsicht auf ein Regalfach mit Fehlbelegung;
Figur 6b einen Vergleich zwischen dem Sollabbild gemäß Figur 2a und dem Istabbild des in Figur 6a gezeigten Regalfaches.

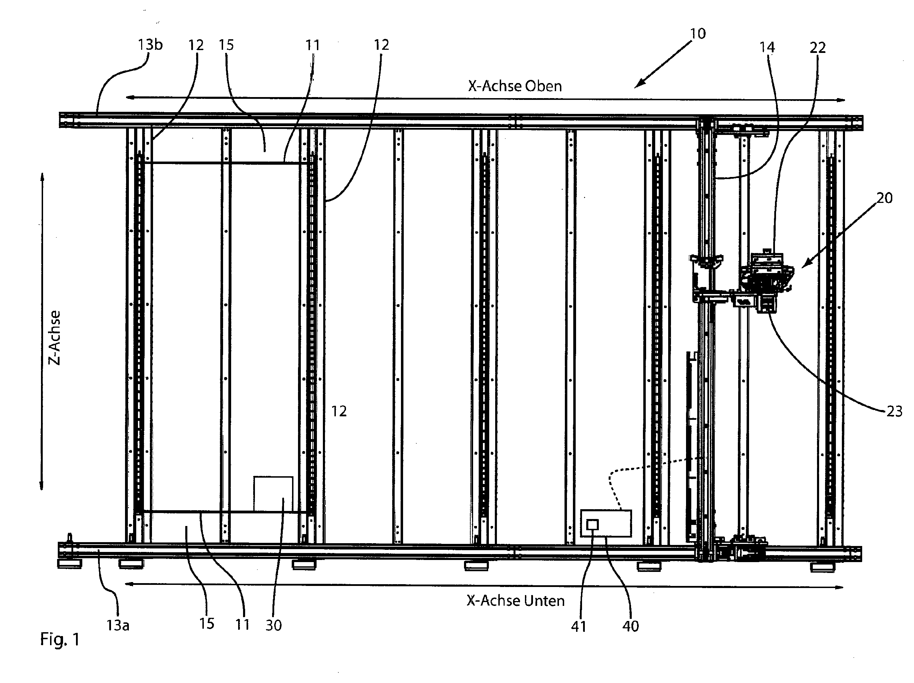

Figur 1 zeigt eine Seitenansicht einer Regalreihe 10 einer Apothekenkommissioniervorrichtung. Die Regalreihe 10 umfasst eine Mehrzahl von senkrechten Regalwänden 12 und eine Mehrzahl von waagerechten Regalböden 11, bei denen es sich üblicherweise um Glasböden oder Böden aus einem vergleichbaren Material handelt. Die Regalböden 11 und die Regalwände 12 bilden jeweils Regalfächer 15, in welchen die Arzneimittelpackungen gelagert werden, wobei in Figur 1 lediglich beispielhaft eine Arzneimittelpackung 30 dargestellt ist.

Vor der Regalreihe 10 ist ein Bediengerät 20, welches einen Sensor 23 und eine Greifvorrichtung 22 umfasst, an einer Führung, welche zwei horizontale Führungsschienen 13a, 13b und eine vertikale Führungsschiene 14 umfasst, verfahrbar. Das Bediengerät 20 ist mit einer Steuereinrichtung 40, die unter anderem einen Speicher 41 aufweist, gekoppelt.

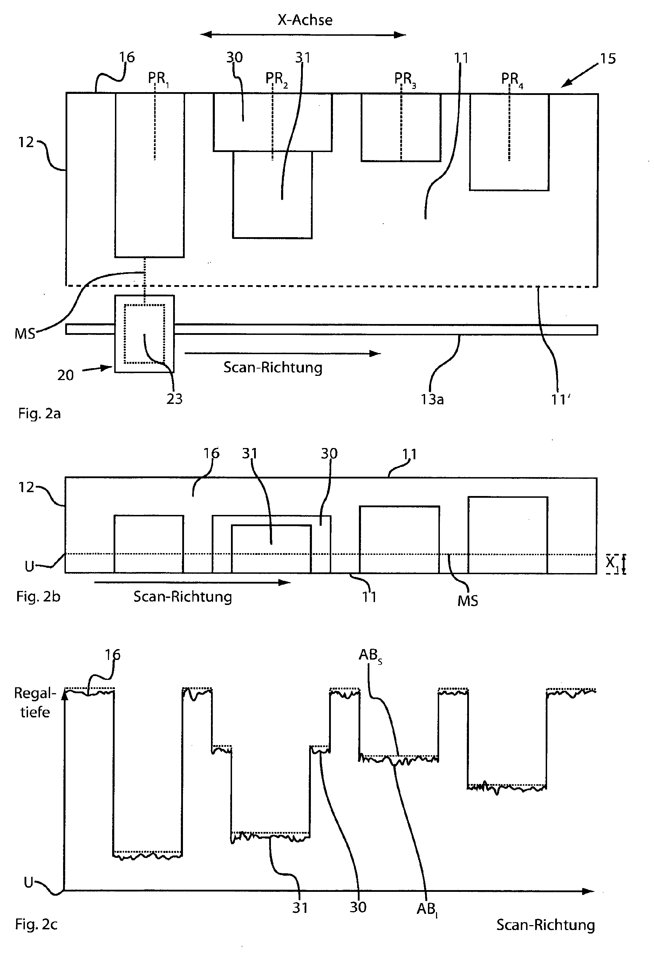

Figur 2a zeigt eine Draufsicht auf ein lediglich schematisch dargestelltes Regalfach 15, welches durch zwei Regalwände 12, eine Regalrückwand 16 und den Regalboden 11 gebildet ist. In der Apothekenkommissioniervorrichtung ist pro Regalreihe 10 eine Mehrzahl solcher Regalreihen angeordnet. Die Abmessungen der einzelnen Fächer sind von dem Gesamtaufbau der Apothekenkommissioniervorrichtung abhängig. Bedingt durch die Art der Lagerung und die vollautomatische Ein- und Auslagerung sind die Regalfächer üblicherweise langgestreckt, wobei die Längsachse der X-Achse der Apothekenkommissioniervorrichtung entspricht. In den Regalfächern sind die Arzneimittelpackungen wiederrum in Reihen gelagert, wobei pro Reihe auch mehrere Arzneimittelpackungen gelagert sein können.

Bei dem gezeigten Ausführungsbeispiel sind in dem Regalfach 15 vier Packungsreihen PR₁ - PR₄ vorhanden, wobei die Packungsreihe PR₂ zwei Arzneimittelpackungen 31, 31 umfasst.

Die Arzneimittelpackungen werden üblicherweise, sofern lediglich eine Arzneimittelpackung pro Packungsreihe angeordnet ist, bündig an der Regalrückwand 16 gelagert. Sind mehrere Arzneimittelpackungen in einer Packungsreihe gelagert, werden diese Arzneimittelpackungen bündig aneinander gelagert.

Zur Ermittlung von Fehllagerungen bei einem Regalfach wird dieses zunächst, beispielsweise von einem Benutzer oder automatisch nach einer händischen Entnahme, als Regalfach mit möglichen Fehllagerungen markiert. Das Bediengerät 20, welches in Figur 2 nicht maßstabsgetreu wiedergegeben ist, wird an eine vorgegebene Regalposition bei dem ermittelten Regalfach verfahren, wobei diese Regalposition üblicherweise links oder rechts außen liegen wird, um das Verfahren möglichst rasch durchführen zu können (sofern, wovon bei der Beschreibung des Verfahrens ausgegangen wird, die Einzelpunktmessung verwendet wird).

Mit eingeschaltetem Sensor 23 wird dann mit dem Bediengerät das Regalfach in horizontaler Richtung vollständig abgefahren. Dabei ist die Bewegungsrichtung unwesentlich. Auch kann das Abfahren des Regales beispielsweise in der Mitte starten, wobei dann jedoch weitere Wege zurückzulegen sind. Wichtig ist lediglich, dass mit dem Sensor das Regal in X-Richtung vollständig abgefahren wird und dabei die Einlagerungstiefe bei verschiedenen X-Positionen ermittelt wird. Wie die Einlagerungstiefen im Einzelnen erfasst werden ist für das erfindungsgemäße Verfahren unwesentlich und bedarf daher keiner detaillierten Beschreibung; wichtig ist, dass bei einer Mehrzahl von Punkten der Abstand Sensor Arzneimittelpackung / Regalrückwand ermittelt wird.

Der Sensor ist dabei selbstverständlich so auszurichten, dass er nicht auf die Stirnseite des Regalbodens oder über einige der Arzneimittelpackungen gerichtet ist, sondern die Stirnfläche sämtlicher in dem Regalfach gelagerten Arzneimittelpackungen erfassen kann, sofern dies aufgrund ihrer Anordnung vor- bzw. hintereinander möglich ist.

Figur 2b zeigt eine Vorderansicht des in Figur 2a gezeigten Regalfachs 15, wobei mit der punktierten Linie der Weg des Messstrahls bei dem Abfahren des Regalfachs veranschaulicht ist. Der Auftreffpunkt des Messstrahls ist so gewählt (Abstand X₁ zum Regalboden 11), dass die Stirnflächen sämtlicher "sichtbarer" Arzneimittelpackungen erfasst werden können. Der Startpunkt bei der Erzeugung des Tiefen- bzw. Abstandsprofils ist mit "U" angedeutet.

Bei dem Abfahren des Regalfaches in X-Richtung wird anhand der X-Positionen und der Einlagerungstiefen ein das Tiefenprofil wiedergebende Istabbild AB_{I} des Regalfaches erstellt, welches schematisch in Figur 2c als gezackte Linie wiedergegeben ist (die gezackte Linie soll Messunsicherheiten andeuten). In Abhängigkeit von der Breite und der Art der eingelagerten Arzneimittelpackungen muss keine, wie dies in Figur 2b dargestellt ist, Dauermessung durchgeführt werden, sondern es kann ausreichend sein, dass die Abstandsmessung lediglich punktuell durchgeführt wird, beispielsweise pro Millimeter jeweils eine Messung. In Figur 3c ist ferner das Sollabbild AB_{S} der theoretischen Belegung des Regalfachs 15, bestimmt aus den gespeicherten Informationen über die einzelnen Arzneimittelpackungen, wiedergegeben. Wie zu erkennen ist, entspricht das Istabbild, abgesehen von Abweichungen basierend auf Messunsicherheiten bzw. -schwankungen, im Wesentlichen dem Sollabbild.

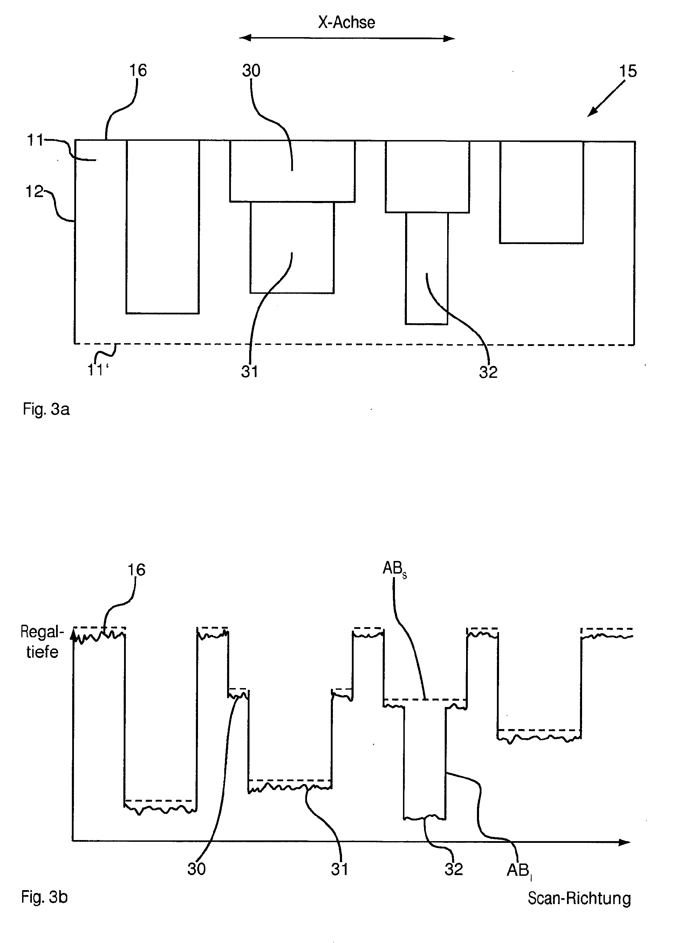

Figur 3a zeigt das Regalfach 15 mit einer Belegung, bei welcher, verglichen mit der Fachbelegung aus Figur 2a, zusätzlich eine Arzneimittelpackung 32 in Packungsreihe PK₃ angeordnet ist.

Die Figur 3b zeigt (als gezackte Linie) das auf der Basis der ermittelten Einlagerungstiefen erhaltene virtuelle Istabbild AB_{I} des gemäß Figur 3a belegten Regalfaches 15. In Figur 3b ist ferner (als punktierte Linie) das Sollabbild AB_{S} der Belegung des in Figur 2a gezeigten Regalfaches wiedergegeben.

Bei dem erfindungsgemäßen Verfahren werden das Sollabbild und das Istabbild miteinander verglichen und Abweichungen zwischen dem Sollabbild und dem Istabbild ausgewertet. Der Vergleich ergibt im vorliegenden Fall, dass das Istabbild bei Packungsreihe PK₃ eine Fläche aufweist, die gemäß dem Sollabbild nicht vorhanden sein sollte. Die Auswertung dieser Abweichung ergibt, dass in dem Regalfach 15 eine Arzneimittelpackung gelagert ist, die dort gemäß der theoretischen Belegung des Regalfaches nicht vorhanden sein sollte.

Diese Arzneimittelpackung 32 wird, um die theoretische Belegung des Regalfaches 15 wiederherzustellen, ausgelagert und dann gegebenenfalls neu identifiziert und eingelagert.

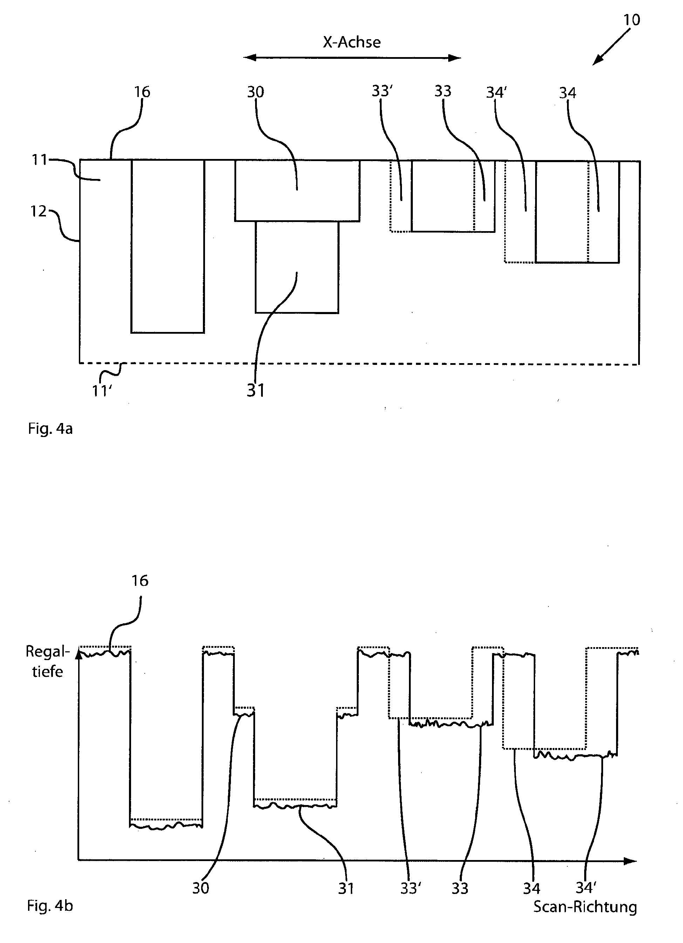

Figur 4a zeigt das Regalfach 15 mit einer Belegung, bei welcher, verglichen mit der Fachbelegung aus Figur 2a, Arzneimittelpackungen 33, 34 in Packungsreihen PK₃ und PK₄ verschoben sind (die ursprünglichen Lagerplätze sind mit 33' und 34' angedeutet).

Die Figur 4b zeigt (als gezackte Linie) das auf der Basis der ermittelten Einlagerungstiefen erhaltene virtuelle Istabbild AB_{I} des gemäß Figur 4a belegten Regalfaches 15. In Figur 4b ist ferner (als punktierte Linie) das Sollabbild AB_{S} der Belegung des in Figur 2a gezeigten Regalfaches wiedergegeben.

Die Auswertung des Vergleichs Sollabbild/Istabbild ergibt, dass zwei Arzneimittelpackungen 33, 34 verschoben sind. Die Auswertung ergibt ferner, dass die Arzneimittelpackungen 34 derart verschoben sind (nämlich um den gleichen Betrag nach "rechts"), dass diese noch mit der Greifvorrichtung 22 gegriffen werden können. Die Arzneimittelpackungen 33, 34 werden dementsprechend mit der Greifvorrichtung wieder an ihren dem theoretischen Bestand entsprechenden Lagerplatz verbracht. Dazu können die Arzneimittelpackungen auf dem Regalboden 11 verschoben oder ausgelagert und erneut eingelagert werden.

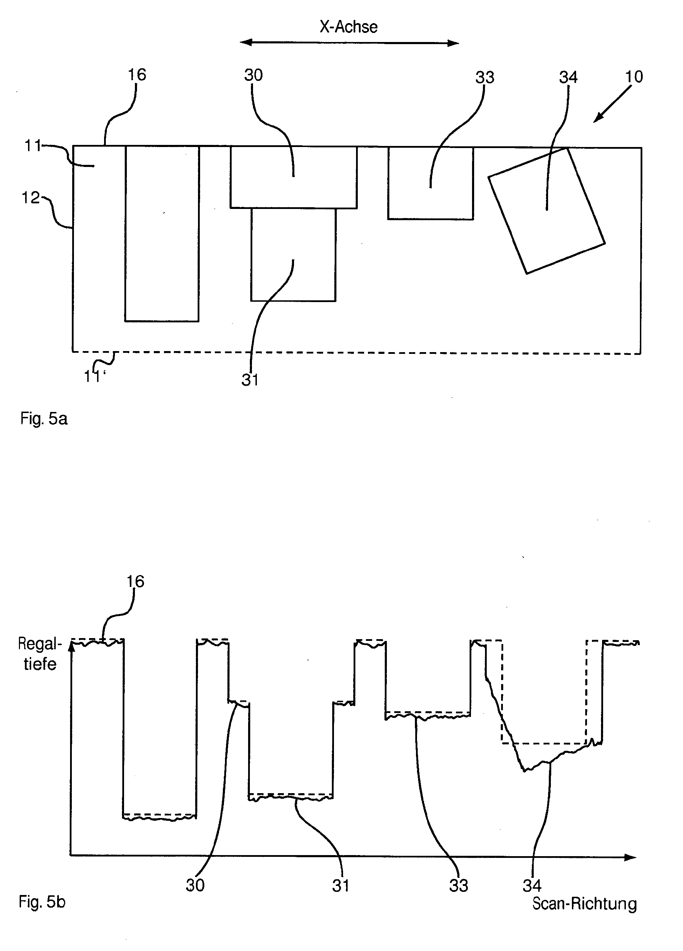

Figur 5a zeigt das Regalfach 15 mit einer Belegung, bei welcher, verglichen mit der Fachbelegung aus Figur 2a, Arzneimittelpackung 34 in Packungsreihe PK₄ verschoben ist, und zwar derart, dass die Arzneimittelpackung nicht mehr an der Regalrückwand 16 anliegt.

Die Figur 5b zeigt (als gezackte Linie) das auf der Basis der ermittelten Einlagerungstiefen erhaltene virtuelle Istabbild AB_{I} des gemäß Figur 5a belegten Regalfaches 15. In Figur 5b ist ferner (als punktierte Linie) das Sollabbild AB_{S} der Belegung des in Figur 2a gezeigten Regalfaches wiedergegeben.

Die Auswertung des Vergleichs Sollabbild/Istabbild ergibt, dass die Arzneimittelpackung 34 verschoben ist. Die Auswertung ergibt ferner, dass die Arzneimittelpackungen 34 trotz der Verschiebung einer theoretisch im Lagerbestand vorhandenen Arzneimittelpackung zugeordnet werden kann und dass diese noch mit der Greifvorrichtung 22 gegriffen werden kann (zwischen der Arzneimittelpackung 34 ist sowohl links als auch rechts genug Abstand zur benachbarten Arzneimittelpackung bzw. Regalwand um eine Auslagerung mit der Greifvorrichtung durchzuführen). Die Arzneimittelpackung 34 wird mit der Greifvorrichtung ausgelagert und erneut eingelagert. Ein bloßes Verschieben auf dem Regalboden ist aufgrund der Schieflage nicht möglich.

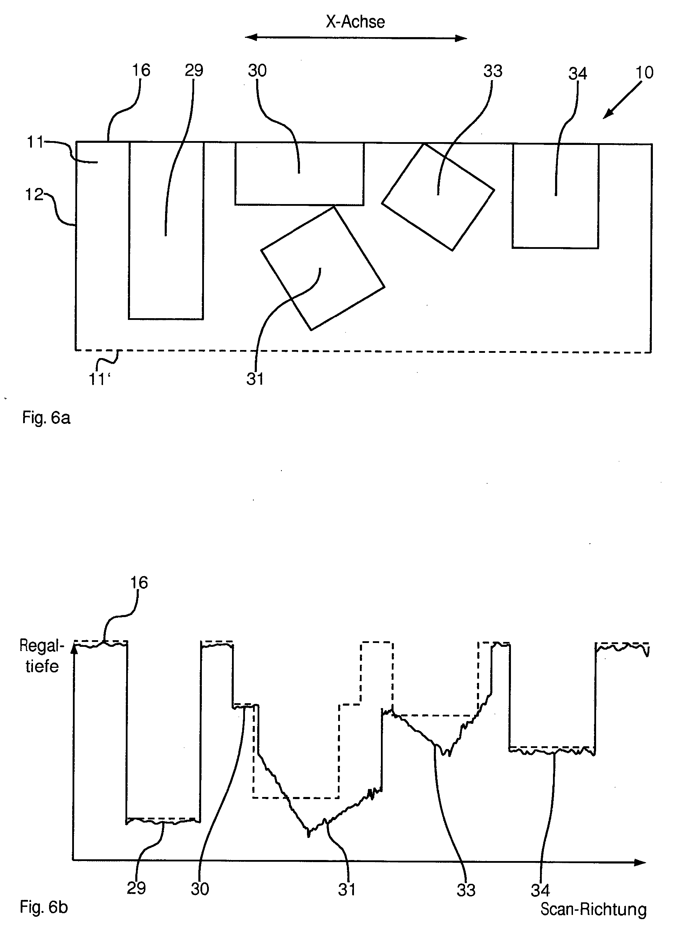

Figur 6a zeigt das Regalfach 15 mit einer Belegung, bei welcher, verglichen mit der Fachbelegung aus Figur 2a, zwei Arzneimittelpackungen 31, 34 in Packungsreihen PK₂ und PK₃ verschoben bzw. verdreht sind.

Die Figur 6b zeigt (als gezackte Linie) das auf der Basis der ermittelten Einlagerungstiefen erhaltene virtuelle Istabbild AB_{I} des gemäß Figur 6a belegten Regalfaches 15. In Figur 6b ist ferner (als punktierte Linie) das Sollabbild AB_{S} der Belegung des in Figur 2a gezeigten Regalfaches wiedergegeben.

Die Auswertung des Vergleichs Sollabbild/Istabbild ergibt, dass die Arzneimittelpackungen 31, 33 verschoben bzw. verdreht sind. Die Auswertung lässt aufgrund der Verdrehung bzw. Verschiebung der Arzneimittelpackung 31 keine eindeutigen Rückschlüsse auf die in derselben Packungsreihe liegende Arzneimittelpackung 30 zu.

Die Packungen können nicht problemlos ausgelagert werden, ohne dass die Gefahr besteht, dass auch umliegende, auf dem theoretisch vorgesehenen Lagerplatz liegende Arzneimittelpackungen, ggf. verschoben werden. Bei dem Istabbild ist nämlich nicht eindeutig zu erkennen, wo die Arzneimittelpackungen 31, 33 enden, da sie sich ihre bei der Erfassung der Einlagerungstiefe für den Sensor erfassbaren Stirnflächen überdecken.

Die Auswertung ergibt ferner, dass die Arzneimittelpackungen 31, 33 (und auch 30) nicht mehr eindeutig theoretisch im Lagerbestand vorhandenen Arzneimittelpackungen zugeordnet werden können. Es kann beispielsweise nicht ausgeschlossen werden, dass bei der händischen Auslagerung die Arzneimittelpackungen 31, 33 vertauscht wurden etc.

Erfindungsgemäß wird in einem solchen Fall geprüft, welche benachbarten Arzneimittelpackungen theoretisch vorhandenen Arzneimittelpackungen zugeordnet werden können (hier Arzneimittelpackungen 29, 34. Dann werden sämtliche oder zumindest ein Teil der Arzneimittelpackungen, die theoretisch vorhandenen zugeordnet werden könne, aus- bzw. umgelagert und dann die verbliebenen Arzneimittelpackungen ausgelagert. Zu diesem Zeitpunkt ist das Regalfach um die Fehlerstelle weiträumig ausgeräumt und die Greifvorrichtung kann die Arzneimittelpackungen bei Bedarf so lange separieren, bis die Arzneimittelpackungen mit der Greifvorrichtung ausgelagert werden können; ggf. muss dazu für die verbliebenen und ggf. separierten Arzneimittelpackungen noch einmal die Einlagerungstiefe und somit ein Istabbild des Regalfachs (oder des betroffenen Abschnitts) ermittelt werden. Nach Auslagerung der Arzneimittelpackungen werden diese identifiziert und dann wieder eingelagert.

## Patentansprüche

1. Verfahren zum Erkennen von Fehllagerungen von Arzneimittelpackungen in einer Apothekenkommissioniervorrichtung mit
zumindest einer Regalreihe mit jeweils einer Mehrzahl von sich in einer horizontalen Richtung (X-Achse) erstreckenden Regalböden (11) und einer Mehrzahl von sich in einer vertikalen Richtung ersteckenden Regalwänden (12), wobei die Regalböden (11) und die Regalwände (12) eine Mehrzahl von Regalfächern (15) bilden,
zumindest einem vor der Regalreihe horizontal und vertikal verfahrbaren Bediengerät (20), wobei das Bediengerät (20) eine Greifvorrichtung (22) zum Ein- und/oder Auslagern von Arzneimittelpackungen (29 - 34) auf die bzw. von den Regalböden (11) sowie einen Sensor (23) umfasst,
und einer mit dem Bediengerät (20) gekoppelten Steuereinrichtung (40), wobei zum Erkennen von Fehllagerungen
Regalfächer (15) mit möglichen Fehllagerungen von Arzneimittelpackungen (29 - 34) ermittelt werden,
das Bediengerät (20) an eine vorgegebene Regalposition bei einem ermittelten Regalfach (15) verfahren wird,
mit dem Bediengerät (20) bei eingeschaltetem Sensor (23) ein ermitteltes Regalfach in horizontaler Richtung (X-Achse) abgefahren und mit dem Sensor (23) die Einlagerungstiefe bei verschiedenen X-Positionen ermittelt wird,
auf der Basis der ermittelten Einlagerungstiefen ein virtuelles Istabbild eines ermittelten Regalfaches (15) erstellt wird,
das erstellte Istabbild (AB_{I}) mit einem Sollabbild (AB_{S}) des Regalfaches (15) verglichen wird, und
Abweichungen zwischen dem Sollabbild und dem Istabbild ausgewertet werden.

2. Verfahren zum Erkennen von Fehllagerungen von Arzneimittelpackungen nach Anspruch 1, wobei Regalfächer (15) mit möglichen Fehllagerungen ermittelt werden, indem diese von einem Benutzer oder automatisch nach einer händischen Auslagerung von Arzneimittelpackungen als Regalfächer mit möglichen Fehllagerungen markiert werden.

3. Verfahren zum Erkennen von Fehllagerungen von Arzneimittelpackungen nach Anspruch 1, wobei Regalfächer (15) mit möglichen Fehllagerungen ermittelt werden, indem nach einer Störung der Apothekenkommisioniervorrichtung betroffene Regalfächer (15) als Regalfächer mit möglichen Fehllagerungen markiert werden.

4. Verfahren zum Erkennen von Fehllagerungen von Arzneimittelpackungen nach einem der Ansprüche 1 - 3, wobei das Sollabbild des Regalfaches (15) für den Vergleich mit dem erstellten Istabbild anhand einer Sollbelegung des Regalfaches (15) errechnet wird.

5. Verfahren zum Erkennen von Fehllagerungen von Arzneimittelpackungen nach einem der Ansprüche 1 - 3, wobei das Sollabbild des Regalfaches (15) beim Ein- und/oder Auslagern von Arzneimittelpackungen in bzw. aus dem Regalfaches (15) erstellt bzw. aktualisiert wird und für den Vergleich aus einem Speicher (41) der Steuereinrichtung (40) geladen wird.

6. Verfahren zum Erkennen von Fehllagerungen von Arzneimittelpackungen nach einem der Ansprüche 1 - 5, wobei, wenn die Auswertung der Abweichungen zwischen dem Sollabbild und dem Istabbild ergibt, dass eine Arzneimittelpackung in dem Regalfach (15) fehlt, diese aus einem in der Steuereinrichtung (40) gespeicherten theoretischen Lagerbestand gelöscht wird.

7. Verfahren zum Erkennen von Fehllagerungen von Arzneimittelpackungen nach einem der Ansprüche 1 - 5, wobei, wenn die Auswertung der Abweichungen zwischen dem Sollabbild und dem Istabbild ergibt, dass eine nicht im theoretischen Lagerbestand verzeichnete Arzneimittelpackung (32) in dem Regalfach (15) gelagert ist, diese ausgelagert oder identifiziert und erneut eingelagert wird.

8. Verfahren zum Erkennen von Fehllagerungen von Arzneimittelpackungen nach einem der Ansprüche 1 - 5, wobei, wenn die Auswertung der Abweichungen zwischen dem Sollabbild und dem Istabbild ergibt, dass eine Arzneimittelpackung (31, 33, 34) verschoben ist,
anhand der Fehllagerung der Arzneimittelpackung geprüft wird, ob die verschobene Arzneimittelpackung mit der Greifvorrichtung (22) ausgelagert werden kann, und
sofern eine Auslagerung mit der Greifvorrichtung (22) möglich ist, diese Arzneimittelpackung ausgelagert und erneut eingelagert wird.

9. Verfahren zum Erkennen von Fehllagerungen von Arzneimittelpackungen nach Anspruch 8, wobei, wenn eine Auslagerung mit der Greifvorrichtung (22) nicht möglich ist,
geprüft wird, ob die Arzneimittelpackung eindeutig einer theoretisch vorhandenen Arzneimittelpackung zugeordnet werden kann, und,
sofern diese der Fall ist, zunächst die umliegenden Arzneimittelpackungen ausgelagert werden und dann die verbliebende Arzneimittel ausgelagert und erneut eingelagert wird.

10. Verfahren zum Erkennen von Fehllagerungen von Arzneimittelpackungen nach Anspruch 9, wobei, wenn die Arzneimittelpackung nicht eindeutig einer theoretisch vorhandenen Arzneimittelpackung zugeordnet werden kann,
ermittelt wird, welche benachbarten Arzneimittelpackungen theoretisch vorhandenen Arzneimittelpackungen zugeordnet werden können,
zunächst sämtliche Arzneimittelpackungen, die theoretisch vorhandenen zugeordnet werden können, ausgelagert werden und dann die verbliebenen Arzneimittelpackungen ausgelagert, erneut identifiziert und dann wieder eingelagert werden.

11. Verfahren zum Erkennen von Fehllagerungen von Arzneimittelpackungen nach einem der Ansprüche 1 - 10, wobei dann, wenn Arzneimittelpackungen nicht mit der Greifvorrichtung ausgelagert werden können, ein Benutzer entsprechend informiert und zur händischen Auslagerung aufgefordert wird.

12. Verfahren zum Erkennen von Fehllagerungen von Arzneimittelpackungen nach einem der Ansprüche 1 - 11, wobei nach einer Bereinigung eines Regalfaches (15) mit möglichen Fehllagerungen dieses bei eingeschaltetem Sensor (23) ein ermitteltes Regalfach in horizontaler Richtung (X-Achse) noch einmal vollständig abgefahren und mit dem Sensor (23) die Einlagerungstiefe bei verschiedenen X-Positionen ermittelt wird, auf der Basis der ermittelten Einlagerungstiefe pro X-Position ein virtuelles Istabbild eines ermittelten Regalfaches (15) erstellt wird, das erstellte Istabbild mit einem Sollabbild des Regalfaches (15) verglichen wird, und Abweichungen zwischen dem Sollabbild und dem Istabbild ausgewertet werden.

## Claims

1. A method of determining the incorrect positioning of medicine packages in a pharmacy order picking system with
at least one row of shelves each having a plurality of shelf bases (11) extending in a horizontal direction (X-axis) and a plurality of shelf walls (12) extending in a vertical direction, whereby the shelf bases (11) and the shelf walls (12) form a plurality of shelf compartments (15),
at least one operating device (20) which can be moved horizontally and vertically in front of the row of shelves, wherein the operating device (20) comprises a gripper device (22) for the putting into and/or taking out of stock of medicine packages (29 - 34) onto or from the shelf bases (11), as well as a sensor (23),
a control system (40) connected to the operating device (20) whereby in order to to determine incorrect positioning
shelf compartments (15) with possibly incorrectly positioned medicine packages (29 - 34) are determined,
the operating device (20) is moved to a predetermined shelf position in the case of a determined shelf compartment (15),
by way of the operating device (20), with the sensor (23) switched on, a determined shelf compartment is scanned in the horizontal direction (X-axis) and with the sensor (23) the stocking depth at various X positions is determined,
on the basis of the determined stocking depths a virtual actual picture of a determined shelf compartment (15) is produced,
the produced actual picture (AB_{A}) is compared with a nominal picture (AB_{N}) of the shelf compartment (15) and
differences between the nominal picture and the actual picture are evaluated.

2. The method of determining the incorrect positioning of medicine packages according to claim 1, wherein shelf compartments (15) with possible incorrect positioning are determined in that these are marked as being shelf compartments with possible incorrect positioning by a user, or automatically after manually taking medicine packages out of stock.

3. The method of determining the incorrect positioning of medicine packages according to claim 1, wherein shelf compartments (15) with possible incorrect positioning are determined in that after a fault in the pharmacy order picking system, shelf compartments (15) that have been affected are marked as being shelf compartments with possible incorrect positioning.

4. The method of determining the incorrect positioning of medicine packages according to any one of claims 1 - 3, whereby the nominal picture of the shelf compartment (15) is determined for comparison with the produced actual picture on the basis of the nominal occupancy of the shelf compartment (15).

5. The method of determining the incorrect positioning of medicine packages according to any one of claims 1 - 3, wherein the nominal picture of the shelf compartment (15) during the putting into and/or taking out of stock of medicine packages into/from the shelf compartment (15) is produced or updated and loaded for comparison from a memory (41) of the control unit (40).

6. The method of determining the incorrect positioning of medicine packages according to any one of claims 1 - 5 wherein, if the evaluation of the differences between the nominal picture and the actual picture shows that a medicine package is missing in this shelf compartment (15), this package is deleted from the theoretical stock saved in the control unit (40).

7. The method of determining the incorrect positioning of medicine packages according to any one of claims 1 - 5, wherein if the evaluation of the differences between the nominal picture and the actual picture shows that a medicine package (32) not listed in the theoretical stock is stored in the shelf compartment (15), it is removed or identified and placed back in stock.

8. The method of determining the incorrect positioning of medicine packages according to any one of claims 1 - 5, wherein if the evaluation of the differences between the nominal picture and the actual picture shows that a medicine package (31, 33, 34) has been displaced,
by way of the incorrect positioning of the medicine package it is checked whether the displaced medicine package can be removed from storage with the gripper device (22), and
if removal from storage with the gripper device (22) is possible, the medicine package is removed from storage and then placed back in stock.

9. The method of determining the incorrect positioning of medicine packages according to claim 8, wherein, if removal from storage with the gripper device (22) is not possible,
it is checked whether the medicine package can be clearly assigned to a theoretically present medicine package, and
it this is the case, the surrounding medicine packages are initially taken out of stock and the remaining medicines are initially taken out stock and then put back in stock again.

10. The method of determining the incorrect positioning of medicine packages according to claim 9, wherein, if the medicine package cannot be clearly assigned to a theoretically present medicine package,
it is determined which adjacent medicine packages can be assigned to theoretically present medicine packages,
initially all medicine packages that can be assigned to those that are theoretically present are taken out of stock and the remaining medicine packages are taken out of stock, newly identified and put back in stock.

11. The method of determining the incorrect positioning of medicine packages according to any one of claims 1 - 10, whereby in the event that medicine packages cannot be taken out of stock by the gripper device, a user is informed accordingly and is requested to remove it from stock manually.

12. The method of determining the incorrect positioning of medicine packages according to any one of claims 1 - 11, wherein after the rectification of a shelf compartment (15) with possible incorrect positioning, with the sensor (23) switched on, this shelf compartment is again fully scanned in the horizontal direction (X-axis) and with the sensor the stocking depth is determined at various X positions, on the basis of the determined stocking depth per X position a virtual actual picture of a determined shelf compartment (15) is produced, the produced actual picture compared with a nominal picture of the shelf compartment (15) and differences between the nominal picture and the actual picture are evaluated.

## Revendications

1. Procédé pour déterminer le mauvais positionnement d'emballages de médicaments dans un dispositif de préparation pharmaceutique, comportant
au moins une série de rayons dotés respectivement d'une pluralité de fonds de rayons (11) s'étendant dans un sens horizontal (axe X) et d'une pluralité de cloisons de rayons (12) s'étendant dans un sens vertical, les fonds de rayons (11) et les cloisons de rayons (12) formant une pluralité de casiers de rayons (15),
au moins un appareil de manoeuvre (20) déplaçable horizontalement et verticalement (20), l'appareil de manoeuvre (20) comprenant un dispositif de préhension (22) pour mettre en stock et/ou sortir de stock des emballages de médicaments (29 - 34) dans les fonds de rayons (11), de même qu'un capteur (23),
et un dispositif de commande (40) couplé à l'appareil de manoeuvre (20), dans lequel, pour détecter des mauvais positionnements,
des casiers de rayons (15) présentant d'éventuels mauvais positionnements d'emballages de médicaments (29 - 34) sont déterminés,
l'appareil de manoeuvre (20) est déplacé jusqu'à une position prédéfinie du rayon dans le cas où un casier de rayon (15) est déterminé,
grâce à l'appareil de manoeuvre (20), le capteur (23) étant activé, un casier de rayon déterminé est déplacé dans le sens horizontal (axe X) et la profondeur de mise en rayon est déterminée pour plusieurs positions X à l'aide du capteur (23),
en partant des profondeurs de mise en rayon déterminées, une représentation réelle d'un casier de rayon déterminé (15) est établie,
la représentation réelle établie (AB_{I}) est comparée à une représentation théorique (AB_{S}) du casier de rayon (15) et
les écarts entre la représentation théorique et la représentation réelle sont évalués.

2. Procédé pour déterminer le mauvais positionnement d'emballages de médicaments selon la revendication 1, dans lequel des casiers de rayons (15) présentant d'éventuels mauvais positionnements sont déterminés par le fait qu'ils sont marqués par un utilisateur ou automatiquement, après une sortie de stock manuelle d'emballages de médicaments, comme casiers de rayons présentant d'éventuels mauvais positionnements.

3. Procédé pour déterminer le mauvais positionnement d'emballages de médicaments selon la revendication 1, dans lequel des casiers de rayons (15) présentant d'éventuels mauvais positionnements sont déterminés par le fait que, après un dysfonctionnement du dispositif de préparation pharmaceutique, les casiers de rayons concernés (15) sont marqués comme casiers de rayons présentant d'éventuels mauvais positionnements.

4. Procédé pour déterminer le mauvais positionnement d'emballages de médicaments selon une des revendications 1 à 3, dans lequel la représentation théorique du casier de rayon (15) est calculée à partir d'une occupation théorique du casier de rayon (15) pour comparaison avec la représentation réelle établie.

5. Procédé pour déterminer le mauvais positionnement d'emballages de médicaments selon une des revendications 1 à 3, dans lequel la représentation théorique du casier de rayon (15) est établie ou actualisée lors de la mise en stock et/ou de la sortie de stock d'emballages de médicaments dans le casier de rayon (15) et chargée depuis une mémoire (41) du dispositif de commande (40) pour faire la comparaison.

6. Procédé pour déterminer le mauvais positionnement d'emballages de médicaments selon une des revendications 1 à 5, dans lequel, si l'évaluation des écarts entre lequel la représentation théorique et la représentation réelle indique qu'un d'emballage de médicament manque dans le casier de rayon (15), il est effacé dans un stock théorique enregistré dans le dispositif de commande (40).

7. Procédé pour déterminer le mauvais positionnement d'emballages de médicaments selon une des revendications 1 à 5, dans lequel, si l'évaluation des écarts entre lequel la représentation théorique et lequel la représentation réelle indique qu'un d'emballage de médicament (32) non enregistré dans le stock théorique est stocké dans le casier de rayon (15), il est sorti de stock ou identifié et remis en stock.

8. Procédé pour déterminer le mauvais positionnement d'emballages de médicaments selon une des revendications 1 à 5, dans lequel, si l'évaluation des écarts entre lequel la représentation théorique et lequel la représentation réelle indique qu'un d'emballage de médicament (31, 33, 34) est décalé,
à partir du mauvais positionnement de l'emballage de médicament, il est vérifié si l'emballage de médicament décalé peut être sorti de stock à l'aide du dispositif de préhension (22) et,
dans la mesure où il est possible de le sortir à l'aide du dispositif de préhension (22), cet emballage de médicament est sorti de stock et remis en stock.

9. Procédé pour déterminer le mauvais positionnement d'emballages de médicaments selon la revendication 8, dans lequel, s'il n'est pas possible de le sortir à l'aide du dispositif de préhension (22),
il est vérifié si l'emballage de médicament peut être associé sans ambiguïté à un emballage de médicament théoriquement existant,
si c'est le cas, d'abord les emballages de médicaments environnants sont sortis de stock puis les médicaments restants sont sortis de stock et remis en stock.

10. Procédé pour déterminer le mauvais positionnement d'emballages de médicaments selon la revendication 9, dans lequel, si l'emballage de médicament ne peut pas être associé sans ambiguïté à un emballage de médicament théoriquement existant,
il est déterminé quels sont les emballages de médicaments voisins qui peuvent être associés aux emballages de médicaments théoriquement existants,
d'abord tous les emballages de médicaments théoriquement existants qui peuvent être associés sont sortis de stock puis les emballages de médicaments restants sont sortis de stock, ré-identifiés puis remis en stock.

11. Procédé pour déterminer le mauvais positionnement d'emballages de médicaments selon une des revendications 1 à 10, dans lequel, si des emballages de médicaments ne peuvent pas être sortis de stock à l'aide du dispositif de préhension, un utilisateur est informé en conséquence et invité à le sortir manuellement de stock.

12. Procédé pour déterminer le mauvais positionnement d'emballages de médicaments selon une des revendications 1 à 11, dans lequel, après vidage d'un casier de rayon (15) présentant d'éventuels mauvais positionnements, le capteur (23) étant activé, un casier de rayon déterminé est à nouveau déplacé complètement dans le sens horizontal (axe X) et la profondeur de mise en stock à diverses positions de X est déterminée à l'aide du capteur (23), une représentation réelle virtuelle d'un casier de rayon déterminé (15) est établie à partir de la profondeur de mise en stock déterminée par position de X, la représentation réelle établie est comparée à une représentation théorique du casier de rayon (15) et les écarts entre la représentation théorique et la représentation réelle sont évalués.
